# EUROPEAN PATENT APPLICATION

(11) **EP 4 442 245 A1**
(43) Date of publication of application: **09.10.2024**
(21) Application number: 22898674.1
(22) Date of filing: 25.11.2022
(51) Int. Cl.: A61K 8/73, A61K 8/31, A61K 8/37, A61K 8/89, A61K 8/891, A61Q 1/00, A61Q 1/12, A61Q 17/04, C08B 3/10, C08B 3/16

(54) **COMPOSITION AND CELLULOSE DERIVATIVE**

(30) Priority: 29.11.2021 JP 2021193177; 03.12.2021 JP 2021196936
(71) Applicant: Daicel Corporation, Osaka-shi, Osaka 530-0011 (JP)
(72) Inventor: MORIMOTO, Hitomi, Tokyo 108-8230 (JP); MORI, Taro, Tokyo 108-8230 (JP); KOYAMA, Hiroshi, Tokyo 108-8230 (JP)
(74) Representative: Grünecker Patent- und Rechtsanwälte PartG mbB
(86) International application number: PCT/JP2022/043619
(87) International publication number: WO 2023/095892

(57) **Abstract**

To provide a cellulose derivative having excellent affinity for various oil agents and a composition containing the cellulose derivative. A composition containing: a cellulose derivative and an oil agent, the cellulose derivative containing a monovalent organic group (L^{a}) that substitutes for at least some of hydrogen atoms constituting hydroxy groups in cellulose, the monovalent organic group (L^{a}) containing a hydrocarbon group having 6 or more carbons at a terminal, the monovalent organic group (L^{a}) containing no ether bond, and an average degree of substitution of the monovalent organic group (L^{a}) being 1.1 or greater. The oil agent is preferably at least one selected from the group consisting of a hydrocarbon oil, an ester oil, and a silicone oil.

## Description

### Technical Field

The present disclosure relates to a composition and a cellulose derivative. More specifically, the present disclosure relates to a composition containing a cellulose derivative and an oil agent, and a cellulose derivative. The present application claims priority to JP 2021-193177 filed in Japan on November 29, 2021 and JP 2021-196936 filed in Japan on December 3, 2021, the contents of which are incorporated herein by reference.

### Background Art

Cellulose is a macromolecular compound in which a large number of glucose units (glucopyranose units) have been polymerized by β-1,4-glucoside bonding. At present, cellulose and derivatives thereof have been widely used for paper, for application onto skin such as a cosmetic material, for a coating agent, for a paint, and for a film forming agent.

For example, Patent Documents 1 and 2 each describe a cosmetic material containing a specific cellulose derivative. Furthermore, Patent Document 3 describes an external preparation for skin containing a specific cellulose derivative. Patent Document 4 describes a film forming agent containing a specific cellulose derivative.

### Citation List

### Patent Document

Patent Document 1: JP 2017-105757 A
Patent Document 2: JP 2007-527861 T
Patent Document 3: JP 8-175929 A
Patent Document 4: JP 2014-12852 A

### Summary of Invention

### Technical Problem

In a case where cellulose or a derivative thereof is used as a component or a raw material for a product, it is important that the cellulose or the derivative thereof exhibits excellent affinity for a solvent, such as solubility, swellability, and dispersibility in the solvent. However, the cosmetic material of Patent Document 1 essentially requires use of a solvent in which specific proportions of a hydrocarbon oil and an ester oil as oil agents are mixed from the perspective of achieving excellent film formability, and there are no descriptions or suggestions for use of a hydrocarbon oil alone or an ester oil alone as an oil agent. The cosmetic composition described in Patent Document 2 contains a nonvolatile oil agent such as wax, and the solubility or swellability of a cellulose derivative for only a volatile oil agent has not been considered. Since the nonvolatile oil agent remains as a film together with the cellulose derivative even after the application, a high affinity is not always achieved. Patent Document 3 describes only an emulsified external preparation for skin. Patent Document 4 only considers the solubility of the cellulose ester in acetone as an organic solvent, and does not consider the solubility in an oil agent.

As described above, for a composition containing a cellulose derivative, Patent Documents 1 to 4 only describe a method that employs emulsification or acetone but no oil agent, or a method that uses a nonvolatile oil agent or a specific mixed solvent as an oil agent. Thus, the range of oil agents that can be used was limited. Therefore, a composition that contains a cellulose derivative and that can use various oil agents has been demanded.

An object of the present disclosure is to provide a cellulose derivative having excellent affinity for various oil agents and a composition containing the cellulose derivative.

### Solution to Problem

As a result of diligent research to solve the issues described above, the inventors of the present disclosure have found that a specific cellulose derivative achieves excellent affinity for various oil agents. The present disclosure relates to inventions completed based on these findings.

That is, the present disclosure provides a composition containing: a cellulose derivative and an oil agent; the cellulose derivative containing a monovalent organic group (L^{a}) that substitutes for at least some of hydrogen atoms constituting hydroxy groups in cellulose, the monovalent organic group (L^{a}) containing a hydrocarbon group having 6 or more carbons at a terminal, and the monovalent organic group (L^{a}) containing no ether bond, and an average degree of substitution of the monovalent organic group (L^{a}) being 1.1 or greater.

The oil agent is preferably at least one selected from the group consisting of a hydrocarbon oil, an ester oil, and a silicone oil.

A content of the hydrocarbon oil is preferably greater than 80 mass%.

A content of the ester oil is preferably greater than 50 mass%.

The cellulose derivative preferably contains a monovalent organic group (L^{c}) having 5 or less carbons, the monovalent organic group (L^{c}) substituting for at least some of hydrogen atoms constituting hydroxy groups in the cellulose, the monovalent organic group (L^{c}) containing a hydrocarbon group at a terminal, and the monovalent organic group (L^{c}) containing no ether bond.

In the cellulose derivative, the average degree of substitution of the monovalent organic group (L^{a}) is preferably greater than or equal to an average degree of substitution of the monovalent organic group (L^{c}).

In the cellulose derivative, the average degree of substitution of the monovalent organic group (L^{c}) is preferably 1.0 or less.

The monovalent organic group (L^{a}) is preferably a group represented by Formula (1):

-X¹-R¹ (1)

In the formula, R¹ represents the hydrocarbon group having 6 or more carbons, X¹ represents a direct bond or a group capable of forming a linking group by bonding to an oxygen atom constituting a hydroxy group in the cellulose. A bond extending to left bonds to an oxygen atom in a cellulose backbone.

The composition is preferably for use in an oil-based cosmetic material.

Furthermore, the present disclosure also provides a cellulose derivative containing a monovalent organic group (L^{b}) containing a group represented by Formula (2) at a terminal:

-O-R² (2)

in the formula, R² represents a hydrocarbon group having 6 or less carbons,
the monovalent organic group (L^{b}) substituting for at least some of hydrogen atoms constituting hydroxy groups in cellulose.

The monovalent organic group (L^{b}) preferably contains 2 or more ether bonds.

The monovalent organic group (L^{b}) preferably bonds to the cellulose backbone through an ester bond having an oxygen atom constituting the hydroxy group as a constituent atom.

The monovalent organic group (L^{b}) preferably contains a (poly)oxyalkylene chain.

A monovalent organic group (L^{a}) substituting for at least some of hydrogen atoms constituting hydroxy groups in the cellulose, the monovalent organic group (L^{a}) containing a hydrocarbon group having 6 or more carbons at a terminal, and the monovalent organic group (L^{a}) containing no ether bond, is preferably contained.

The average degree of substitution of the monovalent organic group (L^{a}) is preferably greater than an average degree of substitution of the monovalent organic group (L^{b}).

The present disclosure also provides a composition containing the cellulose derivative described above and an oil agent.

The present disclosure also provides an oil-based cosmetic material containing the composition.

### Advantageous Effects of Invention

The composition of the present disclosure achieves excellent affinity of the cellulose derivative for oil agents even in a case where various oil agents are used. Thus, for example, even in a case where contents of a hydrocarbon oil and/or an ester oil are high, the cellulose derivative achieves excellent solubility and swellability, and film formability (film forming characteristic) tends to be excellent.

### Brief Description of Drawings

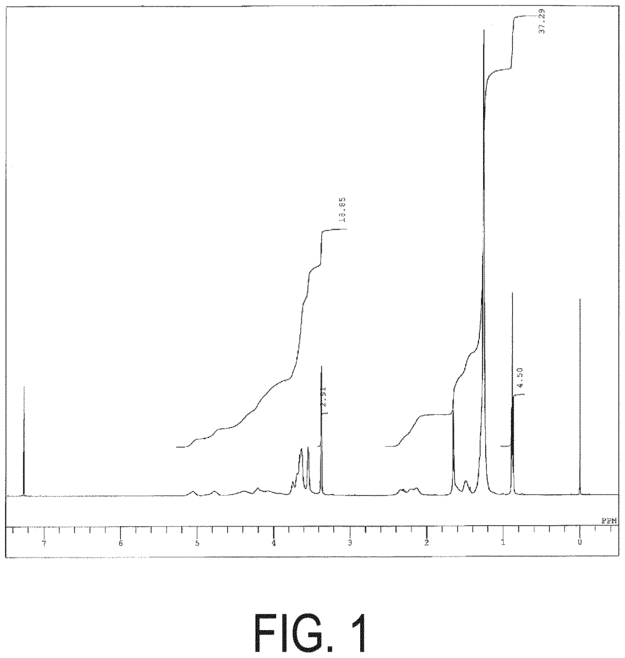

FIG. 1 shows ¹H-NMR spectrum of a cellulose derivative produced in Example 9.

### Description of Embodiments

### Composition

The composition of the present disclosure contains at least a cellulose derivative and an oil agent. The cellulose derivative contains at least a monovalent organic group that substitutes for at least some of hydrogen atoms constituting hydroxy groups in cellulose, the monovalent organic group containing a hydrocarbon group having 6 or more carbons at a terminal, and the monovalent organic group containing no ether bond, the average degree of substitution of the monovalent organic group being 1.1 or greater.

Note that, in the present specification, "cellulose" refers to cellulose that is carbohydrate (polysaccharides) represented by the molecular formula (C₆H₁₀O₅)n. For example, the cellulose described above does not include a substituent substituted with cellulose, such as a hydroxypropyl group in hydroxypropyl cellulose (CAP), a hydroxyethyl group in hydroxyethyl cellulose (HEC), and an acyl group in cellulose acetate butyrate (CAB). The cellulose derivative is specifically a bonded body of a repeating unit represented by Formula (A) below. In the cellulose derivative of the present disclosure, at least one of three groups bonded to any oxygen atom in Formula (A) (i.e., groups bonded to any bond extended from the three oxygen atoms) is a monovalent organic group (L^{a}) or a monovalent organic group (L^{b}). Note that, in the present description, "cellulose backbone" refers to a group other than the group bonded to oxygen atoms derived from the hydroxy groups in the cellulose.

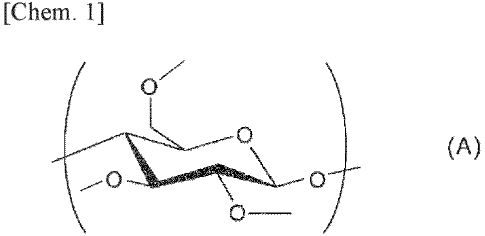

In the present specification, the monovalent organic group containing the hydrocarbon group having 6 or more carbons at a terminal and containing no ether bond may be referred to as a "monovalent organic group (L^{a})".

The monovalent organic group (L^{a}) may substitute for a hydrogen atom of any hydroxy group among three hydroxy groups in the glucose unit in the cellulose. That is, the monovalent organic group (L^{a}) may bond to an oxygen atom of any hydroxy group among the three hydroxy groups in the glucose unit in the cellulose. Furthermore, the cellulose derivative (A) may contain only one type of the monovalent organic group (L^{a}) or two or more types of the monovalent organic groups (L^{a}).

The monovalent organic group (L^{a}) contains a hydrocarbon group having 6 or more carbons at a terminal. When such a monovalent organic group (L^{a}) is contained, the cellulose derivative (A) achieves excellent affinity for various oil agents. The number of carbons in the hydrocarbon group is preferably 7 or greater, more preferably 10 or greater, and even more preferably 12 or greater. Furthermore, the number of carbons in the hydrocarbon group is preferably 24 or less, and more preferably 18 or less. When the number of carbons is 24 or less, tangling of the monovalent organic group (L^{a}) is suppressed, and better affinity for oil agents is achieved.

The hydrocarbon group contained by the monovalent organic group (L^{a}) at a terminal may be in a form of straight chain or branched chain but is preferably a group in a form of straight chain and having 6 or more carbons. Furthermore, the hydrocarbon group may be saturated or unsaturated.

Examples of the hydrocarbon group include a hexyl group, a heptyl group, an octyl group, a 2-ethylhexyl group, a nonyl group, an isononyl group, a decyl group, an undecyl group, a dodecyl group, a lauryl group, a tridecyl group, a tetradecyl group, a myristyl group, a pentadecyl group, a myristyl group, a heptadecyl group, a stearyl group, and an oleyl group.

The monovalent organic group (L^{a}) does not contain an ether bond (-O-). Note that the oxygen atom in the hydroxy group in the cellulose, that is, the oxygen atom linking the monovalent organic group (L^{a}) and the cellulose backbone, is not included in the monovalent organic group (L^{a}). Thus, the monovalent organic group (L^{a}) may be bonded to the cellulose backbone through an ether bond containing an oxygen atom constituting a hydroxy group in the cellulose as a constituent atom. Furthermore, for example, a group in which an organic group is substituted in a hydroxy group in a hydroxypropyl group in CAP through an ether bond does not correspond to the monovalent organic group (L^{a}) since the ether bond derived from the hydroxypropyl group is contained in a part which is not included in the cellulose backbone.

The monovalent organic group (L^{a}) directly bonds to an oxygen atom constituting a hydroxy group in cellulose or bonds to an oxygen atom constituting a hydroxy group in cellulose through a linking group containing the oxygen atom (oxygen atom in the cellulose backbone) as the constituent atom. The linking group is made of the oxygen atom and the monovalent organic group (L^{a}). Examples of the linking group include an ether bond, an ester bond, a urethane bond, a phosphinic acid group, and a thionoester bond. Among them, from the perspective of ease in introduction, an ester bond is preferred.

The monovalent organic group (L^{a}) is preferably a group represented by Formula (1).

-X¹-R¹ (1)

In the formula, R¹ represents the hydrocarbon group having 6 or more carbons, X¹ represents a direct bond or a group capable of forming a linking group by bonding to an oxygen atom constituting a hydroxy group in the cellulose. A bond extending to left bonds to an oxygen atom in a cellulose backbone.

Examples of the linking group that can be formed by X¹ include those described above. Examples of X¹ include a direct bond, a carbonyl group (-C(=O)-), an amide group (-C(=O)-N(-R)-), (-P(=O)(-R)-), and a thiocarbonyl group (-C(=S)-). R is a hydrogen atom or a monovalent organic group. Among these, X¹ is preferably a carbonyl group. That is, the monovalent organic group (L^{a}) is preferably an acyl group.

In the cellulose derivative (A), the average degree of substitution (DS^{a}) of the monovalent organic group (L^{a}) is 1.1 or greater, preferably 1.4 or greater, more preferably 1.7 or greater, even more preferably 2.0 or greater, and particularly preferably 2.2 or greater. When the DS^{a} is 1.1 or greater, the cellulose derivative (A) achieves excellent affinity for various oil agents. The DS^{a} is 3.0 or less, and preferably 2.9 or less, and may be 2.8 or less or 2.7 or less. When the DS^{a} is 2.9 or less, for example, the monovalent organic group (L^{b}) described below can be introduced, and better affinity of the cellulose derivative for oil agents is achieved.

Note that the average degree of substitution in the present description is a number (average value) of groups per repeating unit represented by Formula (A) above in the cellulose derivative. In the present specification, the average degree of substitution can be analyzed and measured by a known or common method, such as ¹H-NMR or ¹³C-NMR. Specifically, for example, calculation can be performed based on an integration ratio of protons in the cellulose backbone to protons in a terminal methyl group of the hydrocarbon group having 6 or more carbons in the monovalent organic group (L^{a}). Furthermore, in a case where a peak of a monovalent organic group contained in the cellulose derivative and a peak derived from the cellulose backbone overlap in an NMR spectrum, a cellulose derivative produced by subjecting hydroxy groups remaining in the cellulose backbone in the cellulose derivative to benzoylation may be analyzed by ¹H-NMR, and calculation may be performed based on an integration ratio of characteristic peaks taking the total average substituent including the benzoyl group as 3.0.

The total number of carbon atoms in the monovalent organic group (L^{a}) is preferably from 6 to 30, and more preferably from 7 to 20. When the total number of carbon atoms is within the range, better affinity of the cellulose derivative for oil agents is achieved.

The cellulose derivative (A) may contain a monovalent organic group (L^{b}) that substitutes for at least some of hydrogen atoms constituting hydroxy groups in cellulose, the monovalent organic group (L^{b}) containing a group represented by Formula (2) below at a terminal. When the monovalent organic group (L^{b}) is contained, packing of the cellulose derivative in an oil agent is suppressed due to the flexibility thereof, and better affinity for oil agents is achieved.

-O-R² (2)

In the formula, R² represents a hydrocarbon group having 6 or less carbons.

R² is a hydrocarbon group having 6 or less carbons. The number of carbons in the hydrocarbon group is preferably 4 or less, and more preferably 2 or less. Examples of the hydrocarbon group include a methyl group, an ethyl group, a propyl group, an isopropyl group, a butyl group, a pentyl group, and a hexyl group.

The hydrocarbon group having the monovalent organic group (L^{b}) at a terminal may be in a form of straight chain or branched chain. Furthermore, the hydrocarbon group may be saturated or unsaturated.

The total number of carbon atoms in the monovalent organic group (L^{b}) is preferably from 1 to 20, and more preferably from 2 to 15. When the total number of carbon atoms is within the range, packing of the cellulose derivative is further suppressed.

The monovalent organic group (L^{b}) preferably contains a (poly)oxyalkylene chain. In this case, packing of the cellulose derivative is further suppressed. The alkylene group in the (poly)oxyalkylene chain is preferably a divalent hydrocarbon group having from 1 to 6 (more preferably from 2 to 4) carbons. Examples of the alkylene group include a methylene group, an ethylene group, a propylene group, a trimethylene group, and a tetramethylene group.

The average degree of polymerization of the oxyalkylene group in the (poly)oxyalkylene chain is preferably from 1 to 5, and more preferably from 2 to 3. When the average degree of polymerization is within the range, packing of the cellulose derivative is further suppressed.

The monovalent organic group (L^{b}) contains at least one ether bond, and preferably contains two or more ether bonds. The number of ether bonds is preferably 6 or less, and more preferably 4 or less. When the average degree of polymerization is within the range, packing of the cellulose derivative is further suppressed.

The monovalent organic group (L^{b}) directly bonds to an oxygen atom in the cellulose backbone or bonds to an oxygen atom in the cellulose backbone through a linking group containing the oxygen atom as the constituent atom. The linking group is made of the oxygen atom and the monovalent organic group (L^{b}). Examples of the linking group include an ether bond, an ester bond, a urethane bond, a phosphinic acid group, and a thionoester bond. Among them, from the perspective of ease in introduction, an ester bond is preferred.

The monovalent organic group (L^{b}) is particularly preferably a group represented by Formula (3).

-X²-R⁴(-O-R³)n-O-R² (3)

In the formula, R² is the same as described above. R³ represents an alkylene group, n represents a degree of polymerization of (-O-R³), R⁴ represents a direct bond or a divalent organic group, X² represents a group capable of forming a linking group by bonding with an oxygen atom constituting a hydroxy group in cellulose. A bond extending to left bonds to an oxygen atom in a cellulose backbone.

(-O-R³)n represents the (poly)oxyalkylene chain. R³ represents an alkylene group, and examples thereof include those exemplified and described as the alkylene group described above. n represents a natural number and is preferably from 1 to 5, and more preferably from 2 to 3.

R⁴ is a direct bond or a divalent organic group. The divalent organic group is preferably a divalent hydrocarbon group. The divalent hydrocarbon group may be saturated or unsaturated, and may be in a form of straight chain or branched chain. The number of carbons of the divalent hydrocarbon group is preferably from 1 to 8. As the divalent hydrocarbon group, an alkylene group, such as a methylene group, an ethylene group, a propylene group, a trimethylene group, and a tetramethylene group, is preferred.

Examples of the linking group that can be formed by X² include those exemplified and described as the linking group that can be formed by X¹ described above. Examples of X² include a carbonyl group (-C(=O)-), an amide group (-C(=O)-N(-R)-), (-P(=O)(-R)-), and a thiocarbonyl group (-C(=S)-). R is a hydrogen atom or a monovalent organic group. Among them, a carbonyl group is preferred.

In the cellulose derivative (A), the average degree of substitution (DS^{b}) of the monovalent organic group (L^{b}) is preferably 0.1 or greater, more preferably 0.5 or greater, and even more preferably 1.0 or greater. When DS^{b} is 0.1 or greater, packing of the cellulose derivative is further suppressed. The DS^{b} is preferably 1.9 or less, more preferably 1.5 or less, and even more preferably 1.3 or less.

In the cellulose derivative (A), the average degree of substitution of the monovalent organic group (L^{a}) is preferably greater than or equal to the average degree of substitution of the monovalent organic group (L^{b}), and more preferably greater than the average degree of substitution of the monovalent organic group (L^{b}). The difference [DS^{a} - DS^{b}] of the average degree of substitution (DS^{a}) of the monovalent organic group (L^{a}) and the average degree of substitution (DS^{c}) of the monovalent organic group (L^{b}) is preferably from 0.1 to 2.5, more preferably from 0.4 to 2.0, and even more preferably from 0.4 to 1.0. When the difference is within the range, better affinity for oil agents is achieved.

The ratio [DS^{a}/DS^{b}] of the average degree of substitution (DS^{a}) of the monovalent organic group (L^{a}) to the average degree of substitution (DS^{b}) of the monovalent organic group (L^{b}) is preferably from 1 to 2.5, and more preferably from 1.3 to 2.2. When the ratio is within the range, better affinity for oil agents is achieved.

The monovalent organic group (L^{b}) may substitute for a hydrogen atom of any hydroxy group among the three hydroxy groups in the glucose unit in the cellulose. Furthermore, the cellulose derivative (A) may contain only one type of the monovalent organic group (L^{b}) or two or more types of the monovalent organic groups (L^{b}).

The cellulose derivative (A) may contain a monovalent organic group (L^{c}) having 5 or less carbons, the monovalent organic group (L^{c}) substituting for at least some of hydrogen atoms constituting hydroxy groups in the cellulose, the monovalent organic group (L^{c}) containing a hydrocarbon group at a terminal, and containing no ether bond. When the monovalent organic group (L^{c}) is contained, better affinity for oil agents tends to be achieved. The hydrocarbon group contained at a terminal of the monovalent organic group (L^{c}) may be saturated or unsaturated, and may be in a form of straight chain or branched chain. Examples of the hydrocarbon group include a methyl group, an ethyl group, a propyl group, an isopropyl group, a butyl group, and a pentyl group.

The monovalent organic group (L^{c}) directly bonds to an oxygen atom in the cellulose backbone or bonds to an oxygen atom in the cellulose backbone through a linking group containing the oxygen atom as the constituent atom. The linking group is made of the oxygen atom and the monovalent organic group (L^{c}). Examples of the linking group include an ether bond, an ester bond, a urethane bond, a phosphinic acid group, and a thionoester bond. Among them, from the perspective of ease in introduction, an ester bond is preferred.

The total number of carbon atoms in the monovalent organic group (L^{c}) is preferably from 1 to 5, and more preferably from 2 to 4.

The monovalent organic group (L^{c}) is particularly preferably a group represented by Formula (4).

-X³-R⁴ (4)

In the formula, R⁴ represents the hydrocarbon group, X³ represents a direct bond or a group capable of forming a linking group by bonding to an oxygen atom in the cellulose. A bond extending to left bonds to an oxygen atom in a cellulose backbone.

Examples of the linking group that can be formed by X³ include those exemplified and described as the linking group that can be formed by X¹ described above. Examples of X³ include a direct bond, a carbonyl group (-C(=O)-), an amide group (-C(=O)-N(-R)-), (-P(=O)(-R)-), and a thiocarbonyl group (-C(=S)-). R is a hydrogen atom or a monovalent organic group. Among these, X³ is preferably a carbonyl group. That is, the monovalent organic group (L^{c}) is preferably an acyl group.

In the cellulose derivative (A), the average degree of substitution of the monovalent organic group (L^{a}) is preferably greater than or equal to the average degree of substitution of the monovalent organic group (L^{c}), and more preferably greater than the average degree of substitution of the monovalent organic group (L^{c}). The difference [DS^{a} - DS^{c}] of the average degree of substitution (DS^{a}) of the monovalent organic group (L^{a}) and the average degree of substitution (DS^{c}) of the monovalent organic group (L^{c}) is preferably from 1.1 to 2.9, and more preferably from 1.4 to 2.7. When the difference is within the range, better affinity for oil agents is achieved.

In the cellulose derivative (A), the average degree of substitution (DS^{c}) of the monovalent organic group (L^{c}) may be 0.05 or greater, or 0.1 or greater. The DS^{c} is preferably 1.0 or less, more preferably 0.5 or less, and even more preferably 0.25 or less.

The ratio [DS^{a}/DS^{c}] of the average degree of substitution (DS^{a}) of the monovalent organic group (L^{a}) to the average degree of substitution (DS^{c}) of the monovalent organic group (L^{c}) is preferably from 5 to 20, and more preferably from 7 to 16. When the ratio is within the range, better affinity for oil agents is achieved.

The monovalent organic group (L^{c}) may substitute for a hydrogen atom of any hydroxy group among the three hydroxy groups in the glucose unit in the cellulose. Furthermore, the cellulose derivative (A) may contain only one type of the monovalent organic group (L^{c}) or two or more types of the monovalent organic groups (L^{c}).

The total of the average degree of substitution (DS^{a}) of the monovalent organic group (L^{a}), the average degree of substitution (DS^{b}) of the monovalent organic group (L^{b}), and the average degree of substitution (DS^{c}) of the monovalent organic group (L^{c}) is 1.1 or greater, preferably 1.5 or greater, more preferably 2.0 or greater, even more preferably 2.5 or greater, and particularly preferably 2.7 or greater. A higher value of the total indicates better affinity for oil agents.

At least some of hydrogen atoms constituting hydroxy groups in the cellulose of the cellulose derivative (A) may be unsubstituted. That is, the cellulose derivative (A) may contain a hydroxy group derived from cellulose. Furthermore, in addition to the monovalent organic group (L^{a}), the monovalent organic group (L^{b}), and the monovalent organic group (L^{c}), the cellulose derivative (A) may contain an additional substituent that substitutes for at least some of hydrogen atoms constituting hydroxy groups in the cellulose. The cellulose derivative (A) may contain only one type of the additional substituent or may contain two or more types of the additional substituents.

In the cellulose derivative (A), the total proportion of at least one substituent selected from the group consisting of the monovalent organic group (L^{a}), the monovalent organic group (L^{b}), and the monovalent organic group (L^{c}) with respect to all the substituents substituting for hydrogen atoms constituting hydroxy groups in the cellulose (100 mol%) is preferably 50 mol% or greater, more preferably 80 mol% or greater, and even more preferably 90 mol% or greater. Furthermore, in the cellulose derivative (A), the proportion of the additional substituent (in particular, a monovalent substituent having a hydroxy group at a terminal) with respect to all the substituents substituting for hydrogen atoms constituting hydroxy groups in the cellulose (100 mol%) is preferably 50 mol% or less, more preferably 20 mol% or less, and even more preferably 10 mol% or less.

As the oil agent, for example, a known or common oil agent that is used in a cosmetic composition can be used, and examples thereof include a hydrocarbon oil, an ester oil, and a silicone oil. The oil agent is preferably a liquid having fluidity at 25°C. The oil agent may be volatile. One type of the oil agent may be used, or two or more types of the oil agents may be used.

The hydrocarbon oil has a flash point of, for example, 35 to 87°C. Examples of the hydrocarbon oil include a paraffin-based hydrocarbon oil, such as n-decane, n-undecane, and n-dodecane, an isoparaffin-based hydrocarbon oil, such as isodecane, isododecane, and hydrogenated polyisobutene, and a cyclic paraffin hydrocarbon oil, such as cyclodecane and cyclododecane.

Examples of the ester oil include isotridecyl isononanoate, diisostearyl malate, isostearyl myristate, octyldodecyl ricinoleate, neopentyl glycol dicaprate, diglyceryl diisostearate, glyceryl monoisostearate monomyristate, 2-ethylhexyl p-methoxycinnamate, tocopherol acetate, and diglyceryl monoisostearate. Note that the ester oil also corresponds to a vegetable oil.

Examples of the silicone oil include octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, and dodecamethylpentasiloxane.

Among these, the oil agent is preferably a hydrocarbon oil or an ester oil.

The content of the cellulose derivative (A) in the composition is preferably from 0.06 to 30 mass%, and more preferably from 0.1 to 20 mass%, with respect to 100 mass% in total of the composition. When the content is 0.06 mass% or greater, better film formability is achieved. When the content is 30 mass% or less, better affinity of the cellulose derivative for oil agents is achieved.

The content of the oil agent in the composition is preferably from 50 to 99.99 mass%, and more preferably from 60 to 99.9 mass%, with respect to 100 mass% in total of the composition. When the content is 50 mass% or greater, better affinity of the cellulose derivative for oil agents is achieved. When the content is 99.99 mass% or less, better film formability is achieved.

In a case where the oil agent contains a hydrocarbon oil as a main component, the content of the hydrocarbon oil in the composition is preferably greater than 50 mass%, more preferably 70 mass% or greater, even more preferably greater than 80 mass%, and particularly preferably 90 mass% or greater, with respect to 100 mass% in total of the composition. Because the cellulose derivative has excellent affinity for various oil agents, even when the content of the hydrocarbon oil is greater than 50 mass% (especially, greater than 80 mass%), excellent affinity of the cellulose derivative for oil agents is achieved.

In a case where the oil agent contains an ester oil as a main component, the content of the ester oil in the composition is preferably greater than 50 mass%, more preferably 70 mass% or greater, even more preferably 80 mass% or greater, and particularly preferably 90 mass% or greater, with respect to 100 mass% in total of the composition. Because the cellulose derivative has excellent affinity for various oil agents, even when the content of the ester oil is greater than 50 mass%, excellent affinity of the cellulose derivative for oil agents is achieved.

The concentration of each component in the composition may be appropriately adjusted by dilution or condensation according to the circumstances during production, during use, and the like.

The composition may contain an additional component besides the cellulose derivative (A) and the oil agent. Examples of the additional component include powder, a surfactant, a lower alcohol, a polyhydric alcohol, a macromolecular compound other than the cellulose derivative (A), an ultraviolet absorber, an antioxidant, a dye, a perfume, a coloring material, an antifoulant, a humectant, and water. One type of the additional component may be used, or two or more types of the additional components may be used. The content of the oil agent in the organic solvent contained in the composition is preferably 80 mass% or greater, more preferably 90 mass% or greater, even more preferably 95 mass% or greater, and particularly preferably 98 mass% or greater, with respect to 100 mass% in total of the organic solvent.

The form of the composition may be any of solid, semi-solid, gel, or liquid.

The composition described above containing the cellulose derivative (A) can be produced by dispersing/dissolving the cellulose derivative (A) in the solvent, or by allowing the cellulose derivative (A) to be swollen with the solvent to form gel.

### Cellulose Derivative

The present disclosure also provides a cellulose derivative containing a monovalent organic group (L^{b}) that substitutes for at least some of hydrogen atoms constituting hydroxy groups in cellulose, the monovalent organic group (L^{b}) containing a group represented by Formula (2) at a terminal.

-O-R² (2)

In the formula, R² represents a hydrocarbon group having 6 or less carbons.

Note that, in the present specification, a cellulose derivative containing at least the monovalent organic group (L^{b}) may be referred to as "cellulose derivative (B)". The cellulose derivative (B) is a novel compound and tends to be excellent in packing suppression.

Examples of the monovalent organic group (L^{b}) in the cellulose derivative (B) include those exemplified and described as the monovalent organic group (L^{b}) that may be contained in the cellulose derivative (A) described above. Preferred aspects of the monovalent organic group (L^{b}) in the cellulose derivative (B) are the same as those of the monovalent organic group (L^{b}) that may be contained in the cellulose derivative (A).

In the cellulose derivative (B), the average degree of substitution (DS^{b}) of the monovalent organic group (L^{b}) is preferably 0.1 or greater, more preferably 0.5 or greater, and even more preferably 1.0 or greater. When DS^{b} is 0.1 or greater, packing of the cellulose derivative is further suppressed. DS^{b} may be 2.9 or less, 2.5 or less, 1.9 or less, 1.5 or less, or 1.3 or less.

The cellulose derivative (B) may contain the monovalent organic group (L^{a}) or may contain no monovalent organic group (L^{a}). Furthermore, the cellulose derivative (B) may contain the monovalent organic group (L^{c}) or may contain no monovalent organic group (L^{c}). At least some of hydrogen atoms constituting hydroxy groups in the cellulose of the cellulose derivative (B) may be unsubstituted. That is, the cellulose derivative (B) may contain a hydroxy group derived from cellulose.

Furthermore, in addition to the monovalent organic group (L^{a}), the monovalent organic group (L^{b}), and the monovalent organic group (L^{c}), the cellulose derivative (B) may contain an additional substituent that substitutes for at least some of hydrogen atoms constituting hydroxy groups in the cellulose. The cellulose derivative (B) may contain only one type of the additional substituent or may contain two or more types of the additional substituents.

Preferred aspects of the cellulose derivative (B) are the same as the preferred aspects of the cellulose (A).

The composition containing the cellulose derivative (B) can be produced by dispersing/dissolving the cellulose derivative (B) in the solvent, or by swelling the cellulose derivative (B) with the solvent to form gel. As the solvent, an oil agent is preferably used.

As the oil agent, a known or common oil agent can be used, and examples thereof include a hydrocarbon oil, an ester oil, and a silicone oil. The oil agent is preferably a liquid having fluidity at 25°C. Among these, the oil agent is preferably a hydrocarbon oil or an ester oil. Examples of the silicone oil, the ester oil, and the hydrocarbon oil include those exemplified and explained as the oil agents in the composition containing the cellulose derivative (A) described above. One type of the oil agent may be used, or two or more types of the oil agents may be used.

The content of the cellulose derivative (B) in the composition is preferably from 0.06 to 30 mass%, and more preferably from 0.1 to 20 mass%, with respect to 100 mass% in total of the composition. When the content is 0.06 mass% or greater, better film formability is achieved. When the content is 30 mass% or less, better affinity of the cellulose derivative for oil agents is achieved.

The content of the oil agent in the composition is preferably from 50 to 99.99 mass%, and more preferably from 60 to 99.9 mass%, with respect to 100 mass% in total of the composition. When the content is 50 mass% or greater, better affinity of the cellulose derivative for oil agents is achieved. When the content is 99.99 mass% or less, better film formability is achieved.

In a case where the oil agent contains a hydrocarbon oil as a main component, the content of the hydrocarbon oil in the composition is preferably greater than 50 mass%, more preferably 70 mass% or greater, even more preferably greater than 80 mass%, and particularly preferably 90 mass% or greater, with respect to 100 mass% in total of the composition. Because the cellulose derivative has excellent affinity for various oil agents, even when the content of the hydrocarbon oil is greater than 50 mass% (especially, greater than 80 mass%), excellent affinity of the cellulose derivative for oil agents is achieved.

In a case where the oil agent contains an ester oil as a main component, the content of the ester oil in the composition is preferably greater than 50 mass%, more preferably 70 mass% or greater, even more preferably 80 mass% or greater, and particularly preferably 90 mass% or greater, with respect to 100 mass% in total of the composition. Because the cellulose derivative has excellent affinity for various oil agents, even when the content of the ester oil is greater than 50 mass%, excellent affinity of the cellulose derivative for oil agents is achieved.

The concentration of each component in the composition may be appropriately adjusted by dilution or condensation according to the circumstances during production, during use, and the like.

The composition may contain an additional component besides the cellulose derivative (B) and the oil agent. Examples of the additional component include those exemplified and explained as the additional component in the composition containing the cellulose derivative (A) described above. One type of the additional component may be used, or two or more types of the additional components may be used.

The form of the composition may be any of solid, semi-solid, gel, or liquid.

### Method for Producing Cellulose Derivative

For the cellulose derivative (A) and the cellulose derivative (B), examples include (i) a method of reacting cellulose and an acyl group donor in an ionic liquid, and (ii) a method of reacting cellulose and an acylation donor in a highly polar solvent (dimethylacetamide) in the presence of lithium chloride. For each of the various components such as the ionic liquid, the acyl group donor, and the highly polar solvent, one type may be used alone, or two or more types may be used.

The ionic liquid used in (i) is used as a solvent to dissolve the cellulose and functions as a strong organocatalyst. Only one type of the ionic liquid may be used, or two or more types of the ionic liquids may be used.

The constituent anion of the ionic liquid is not particularly limited, and examples thereof include carboxylic acid-based anions such as formic acid anion, acetic acid anion, and propionic acid anion, PF₆⁻ ion, CF₃SO₃⁻ ion, (CF₃SO₂)₂N⁻ ion, Cl⁻ ion, and BF₄⁻ ion. In a case where a carboxylic acid-based anion is used as the anion, a substituent derived from the carboxylic acid-based anion can be introduced into the cellulose backbone by the method of (i). For example, in a case where acetic acid anion is used as the anion, an acetyl group can be introduced into the cellulose derivative as the monovalent organic group (L^{c}).

The constituent cation of the ionic liquid is preferably an organic cation, and examples thereof include an ammonium cation and a heterocyclic onium cation. Examples of the ammonium cation include aliphatic quaternary ammonium ions such as trimethylpropylammonium ion, trimethylhexylylammonium ion, tetrapentylammonium ion, and diethyltrimethyl(2-methoxyethyl)ammonium ion, and alicyclic quaternary ammonium ions such as N-butyl-N-methylpyrrolidinium ion.

Examples of the heterocyclic onium cation include an imidazolium cation, a pyridinium cation, a piperidinium cation, and a pyrrolidinium cation. Examples of the imidazolium cation include dialkylimidazolium cations such as 1-ethyl-3-methylimidazolium ion, 1-butyl-3-methylimidazolium ion, and 1-propyl-3-methylimidazolium ion, trialkylimidazolium cations such as 1-(1,2, or 3-hydroxypropyl)-3-methylimidazolium ion, 1,2,3-trimethylimidazolium ion, 1,2-dimethyl-3-propylimidazolium ion, and 1-butyl-2,3-dimethylimidazolium ion, 1-allyl-3-alkylimidazolium cations such as 1-allyl-3-ethylimidazolium ion and 1-allyl-3-butylimidazolium ion, and 1,3-diallylimidazolium cations. Examples of the pyridinium cation include N-propylpyridinium ion, N-butylpyridinium ion, 1-butyl-4-methylpyridinium ion, and 1-butyl-2,4-dimethylpyridinium ion. Examples of the piperidinium cation include N-methyl-N-ethylpiperidinium ion, N-methyl-N-propylpiperidinium ion, and N-methyl-N-butylpiperidinium ion. Examples of the pyrrolidinium cation include N-methyl-N-ethylpyrrolidinium ion, N-methyl-N-propylpyrrolidinium ion, and N-methyl-N-butylpyrrolidinium ion.

The acyl group donor is, for example, a compound that reacts with a hydroxy group in the cellulose and that can substitute an acyl group for a hydrogen atom, and can be appropriately selected depending on the monovalent organic group (L^{a}) or the monovalent organic group (L^{b}). Examples of such a compound include a chain or cyclic ester (e.g., vinyl carboxylate), an aldehyde, a carboxylic halide, and a carboxylic anhydride. Examples of the carboxylic halide include a fluoride, a chloride, a bromide, and an iodide.

The used amount of the acyl group donor is not particularly limited and can be appropriately adjusted based on the degree of substitution of the substituent to be introduced. The used amount of the acyl group donor is, for example, from 100 to 6000 parts by mass, and preferably from 100 to 3000 parts by mass, with respect to 100 parts by mass in total of the cellulose. Furthermore, the used amount of the acyl group donor is, for example, from 1 to 20 equivalents, and preferably from 3 to 9 equivalents, with respect to 1 equivalent of glucose in the cellulose (i.e., 3 equivalents of hydroxy group).

In the reaction of (i), a solvent other than the ionic liquid may be added. As the solvent, a known or common organic solvent or water can be used. The production cost can be reduced while affinity of the cellulose for oil agents is maintained. As the solvent, specifically, the highly polar solvent described above is preferred.

The reaction temperature is, for example, from 40 to 120°C, and the reaction time is, for example, from 1 minute to 48 hours. When the solution after the reaction is subjected to reprecipitation, filtration, and the like by using a solvent such as methanol, the acyl group donor is introduced onto an oxygen atom of a hydroxy group in the cellulose, resulting in the formation of the cellulose derivative containing the monovalent organic group (L^{a}), the monovalent organic group (L^{b}), or the monovalent organic group (L^{c}). Furthermore, the ionic liquid used in the reaction can be recovered for reuse.

The highly polar solvent used in (ii) is used as a solvent to dissolve cellulose. Examples of the highly polar solvent include amide-based solvents such as dimethylformamide, N,N-dimethylacetamide, N-methylpiperidone, and 1,3-dimethyl-2-imidazolidinone; dimethylsulfoxide; sulfolane; γ-butyllactone; and hexamethylphosphoric triamide. Among these, from the perspective of excellent solubility in cellulose, an amide-based solvent is preferred, and N,N-dimethylacetamide is more preferred.

Examples of the acyl group donor include those exemplified and described as the acyl group donor to be used in (i) described above. The used amount of the acyl group donor is not particularly limited and can be appropriately adjusted based on the degree of substitution of the substituent to be introduced. The used amount of the acyl group donor is, for example, from 100 to 6000 parts by mass, and preferably from 100 to 3000 parts by mass, with respect to 100 parts by mass in total of the cellulose. Furthermore, the used amount of the acyl group donor is, for example, from 1 to 20 equivalents, and preferably from 3 to 15 equivalents, with respect to 1 equivalent of glucose in the cellulose (i.e., 3 equivalents of hydroxy group).

The used amount of the lithium chloride is not particularly limited and, for example, from 1 to 12 parts by mass, and preferably from 3 to 9 parts by mass, with respect to 100 parts by mass in total of the highly polar solvent.

In (ii), use of a basic catalyst is preferred. Examples of the basic catalyst include tertiary amines such as triethylamine, pyridine, dimethylaminopyridine, and triphenylphosphine.

The reaction temperature is, for example, from 40 to 120°C, and the reaction time is, for example, from 1 minute to 48 hours. When the solution after the reaction is subjected to reprecipitation, filtration, and the like by using a solvent such as methanol, the acyl group donor is introduced onto an oxygen atom of a hydroxy group in the cellulose, resulting in the formation of the cellulose derivative containing the monovalent organic group (L^{a}), the monovalent organic group (L^{b}), or the monovalent organic group (L^{c}).

### Use of Composition

The composition (composition containing the cellulose derivative (A) and/or the cellulose derivative (B)) achieves excellent affinity of the cellulose derivative for oil agents even when the composition is used for various oil agents. Thus, for example, even in a case where contents of a hydrocarbon oil and/or an ester oil are high, the cellulose derivative achieves excellent affinity for oil agents, and film formability (film forming characteristic) tends to be excellent. Even in a case where the content of the hydrocarbon oil is high, excellent affinity of the cellulose derivative for oil agents is achieved, and thus the content of the ester oil can be made small, and stickiness of a film formed from the composition is also suppressed. Furthermore, because the cellulose derivative can be produced by using cellulose as a raw material and there is no need to be produced through CAP or CAB, a synthesis process of the cellulose derivative can be shortened, which leads to reduction in environmental load and production costs.

The composition can be used in various fields such as packaging paper field, printing field, fiber field, medical field, and cosmetic field. For example, the composition is used for a cosmetic material, an external preparation for skin, a coating agent, and a paint. In the use described above, the composition may be used as a film-forming agent.

The application target of the cosmetic material (cosmetic composition) include skin such as skin, lips, and nails, and hair such as eyelashes and hair. Specific examples of the cosmetic material include lip cosmetics such as lip stick, lip gloss, and lip liner; makeup cosmetics such as mascara, eye liner, eye shadow, cheek color, foundation (e.g., cream foundation, pressed foundation, liquid foundation), and concealer; cream, milky lotion, lotion, all-in-one gel, beauty lotion, facial soap, solid soap, a massage agent, deodorant, sun block, sunscreen, a hair growing agent, shampoo, conditioner, hair colorant, hair oil, hair wax, hair styling spray, hair foam, and a waterproof cosmetic material. The cosmetic material may be in a form of liquid, semi-solid, or solid at room temperature. The cosmetic material is preferably an oil-based cosmetic material containing an oil agent as a base.

The cosmetic material may be filled in a container. Examples of the container include a bottle container, ajar container, and a tube container.

The coating agent can be used for an automobile, wood, plastic, and a metal. Furthermore, in the field of printing, for example, the coating agent can be used for solvent casting of a film. Examples of such use include a film for photography and a protective film for a liquid crystal display.

The medical field described above include for drug delivery. In the use for drug delivery, the cellulose derivative can act as a film forming agent, such as a coating agent for a tablet or a particle. The cellulose derivative can be also used for forming an amorphous mixture for a drug having poor solubility, and thus the solubility and use efficiency in a body of the drug can be improved. The cellulose derivative can be also used in controlled drug delivery, and in this case, the drug can be released from the cellulose derivative responding to exogenous stimulus, such as change in pH.

Each aspect disclosed in the present specification can be combined with any other feature disclosed herein. Note that each of the configurations, combinations thereof, or the like in each of the embodiments are examples, and additions, omissions, replacements, and other changes to the configurations may be made as appropriate without departing from the spirit of the present disclosure. In addition, each aspect of the invention according to the present disclosure is not limited by the embodiments or the following examples but is limited only by the claims.

### Examples

An embodiment of the present disclosure will be described in detail below based on Examples.

### Example 1

In a nitrogen atmosphere, in a mixed solvent of 29 g of ethyl methylimidazolium acetate and 38 g of dimethyl sulfoxide, 1.5 g (9.4 mmol) of microcrystalline cellulose (Avicel, available from Asahi Kasei Corp.) was added and dissolved at 80°C. To this, 8.1 g (36 mmol) of vinyl laurate was added, then allowed to react at 80°C for 30 minutes, purified by allowing the resultant to precipitate in methanol, and dried. Thus, a cellulose derivative in which acetyl groups and lauroyl groups were present as substituents on oxygen atoms in the cellulose backbone (cellulose acetate-laurate) was prepared. The resulting cellulose derivative was heated to 70°C in isododecane, and thus swollen gel of the cellulose derivative (concentration: 10 mass%) was prepared.

### Example 2

In a nitrogen atmosphere, in a mixed solvent of 19 g of ethyl methylimidazolium acetate and 24 g of dimethyl sulfoxide, 1.0 g (6.2 mmol) of microcrystalline cellulose (Avicel, available from Asahi Kasei Corp.) was added and dissolved at 80°C. To this, 4.8 g (18 mmol) of vinyl myristate was added, then allowed to react at 80°C for 40 minutes, purified by allowing the resultant to precipitate in methanol, and dried. Thus, a cellulose derivative in which acetyl groups and myristoyl groups were present as substituents on oxygen atoms in the cellulose backbone (cellulose acetate-myristate) was prepared. The resulting cellulose derivative was heated to 70°C in isododecane, and thus swollen gel of the cellulose derivative (concentration: 10 mass%) was prepared.

### Example 3

3.9 g of a cellulose derivative in which acetyl groups and palmitoyl groups were present as substituents on oxygen atoms in the cellulose backbone (cellulose acetate-palmitate) was prepared in the same manner as in Example 2 except for adding 5.8 g (20 mmol) of vinyl palmitate in place of vinyl myristate and reacting at 80°C for 30 minutes. The resulting cellulose derivative was heated to 70°C in isododecane, and thus swollen gel of the cellulose derivative (concentration: 10 mass%) was prepared.

### Example 4

A cellulose derivative in which acetyl groups and stearoyl groups were present as substituents on oxygen atoms in the cellulose backbone (cellulose acetate-stearate) was prepared in the same manner as in Example 2 except for adding 4.2 g (13 mmol) of vinyl stearate in place of vinyl myristate. The resulting cellulose derivative was heated to 70°C in isododecane, and thus swollen gel of the cellulose derivative (concentration: 10 mass%) was prepared.

### Example 5

A cellulose derivative (cellulose acetate-stearate) was prepared in the same manner as in Example 4 except for changing the added amount of the vinyl stearate to 5.4 g (17 mmol) and reacting at 80°C for 30 minutes. The resulting cellulose derivative was heated to 70°C in isododecane, and thus swollen gel of the cellulose derivative (concentration: 10 mass%) was prepared.

### Example 6

A cellulose derivative (cellulose acetate-stearate) was prepared in the same manner as in Example 5 except for changing the added amount of the vinyl stearate to 7.3 g (23 mmol). The resulting cellulose derivative was added to isododecane and dissolved at 70°C, and thus a cellulose derivative solution (concentration: 10 mass%) was prepared.

### Example 7

In a nitrogen atmosphere, 0.50 g (3.1 mmol) of microcrystalline cellulose (available from Sigma-Aldrich) was added and dissolved in 21.43 g (concentration: 6.7 mass%) of a dimethylacetamide solution in which lithium chloride was dissolved. To this, 1.13 g (9.3 mmol) of dimethylaminopyridine and 4.51 g (27.8 mmol) of octanoyl chloride were added, and then a reaction was performed by agitation at 80°C for 3 hours. Then, a large amount of methanol was added to separate a product, and the product was recovered by filtration. The resulting product was dissolved in 20g of tetrahydrofuran (THF) and purified by being dropped in a large amount of methanol, the precipitate was recovered by filtration and dried, resulting in the formation of 1.01 g of a cellulose derivative (cellulose octanoate) in which octanoyl groups were present as substituents on oxygen atoms in the cellulose backbone. The resulting cellulose derivative was added to isododecane and dissolved at 70°C, and thus a cellulose derivative solution (concentration: 10 mass%) was prepared.

### Example 8

1.64 g of a cellulose derivative in which lauroyl groups were present as substituents on oxygen atoms in the cellulose backbone (cellulose laurate) was prepared in the same manner as in Example 7 except for adding 6.07 g (27.8 mmol) of lauroyl chloride in place of octanoyl chloride. The resulting cellulose derivative was added to isododecane and dissolved at 70°C, and thus a cellulose derivative solution (concentration: 10 mass%) was prepared.

### Preparation Example 1

In a nitrogen atmosphere, thionyl chloride (14.69 g, 0.12 mol) was slowly added dropwise in [2-(2-methoxyethoxy)ethoxy]acetic acid (20.00 g, 0.11 mol) at room temperature. After completion of the dropwise addition, a reaction was performed by agitating at approximately 40°C for approximately 3 hours, then the temperature was increased to 60°C, and thus the reaction was completed. After it was confirmed that by GC analysis the raw materials disappeared, the resultant was cooled to room temperature, and excessive thionyl chloride was subjected to removal by solvent distillation, resulting in the formation of an acid chloride having a hydrophilic group (21.43 g, 0.11 mol, concentration: 97.1 mass%).

### Example 9

In a nitrogen atmosphere, 0.80 g (4.9 mmol) of TENCEL (trade name) (available from Fujibo Holdings, Inc.) was added and dissolved in 32.22 g (concentration: 6.7 mass%) of a dimethylacetamide solution in which lithium chloride was dissolved. To this, a liquid mixture of 1.81 g (14.8 mmol) of dimethylaminopyridine, 14.37 g (22.2 mmol) of the acid chloride prepared in Preparation Example 1, and 4.86 g (22.2 mmol) of lauroyl chloride was added, and then a reaction was performed by agitation at 80°C for 3 hours. Then, a large amount of methanol was added to separate a product, and the product was recovered by filtration. The resulting product was dissolved in 32 g of THF and purified by being dropped in a large amount of methanol, the precipitate was recovered by filtration and dried, resulting in the formation of 3.370 g of a cellulose derivative in which lauroyl groups and groups derived from the acid chloride (may be referred to as "ether bond-containing group") were present as substituents on oxygen atoms in the cellulose backbone. The resulting cellulose derivative was added to isododecane and dissolved at 70°C, and thus a cellulose derivative solution (concentration: 10 mass%) was prepared. The ¹H-NMR spectrum of a cellulose derivative is shown in FIG. 1.

### Example 10

In a nitrogen atmosphere, 0.40 g (2.5 mmol) of microcrystalline cellulose (available from Sigma-Aldrich) was added and dissolved in 16.11 g (concentration: 6.7 mass%) of a dimethylacetamide solution in which lithium chloride was dissolved. To this, a liquid mixture of 0.90 g (7.4 mmol) of dimethylaminopyridine, 2.18 g (11.1 mmol) of the acid chloride prepared in Preparation Example 1, and 2.43 g (11.1 mmol) of lauroyl chloride was added, and then a reaction was performed by agitation at 80°C for 3 hours. Then, a large amount of methanol was added to separate a product, and the product was recovered by filtration. The resulting product was dissolved in 32 g of THF and purified by being dropped in a large amount of methanol, the precipitate was recovered by filtration and dried, resulting in the formation of 0.997 g of a cellulose derivative in which lauroyl groups and groups derived from the acid chloride (ether bond-containing group) were present as substituents on oxygen atoms in the cellulose backbone. The resulting cellulose derivative was added to isododecane and dissolved at 100°C, and thus a cellulose derivative solution (concentration: 10 mass%) was prepared.

### Example 11

The cellulose derivative prepared in Example 1 was added to isotridecyl isononanoate and dissolved at 70°C, and thus a cellulose derivative solution (concentration: 10 mass%) was prepared.

### Comparative Example 1

Cellulose acetate (available from Daicel Corporation) was added to isododecane, and thus a cellulose derivative liquid mixture (mixed proportion: 10 mass%) was prepared.

### Comparative Example 2

A cellulose derivative in which acetyl groups and lauroyl groups were present as substituents on oxygen atoms in the cellulose backbone (cellulose acetate-laurate) was prepared in the same manner as in Example 2 except for adding 1.3 g (5.7 mmol) of vinyl laurate in place of vinyl myristate and reacting at 80°C for 30 minutes. The resulting cellulose derivative was added to isododecane, and thus a cellulose derivative liquid mixture (mixed proportion: 10 mass%) was prepared.

### Comparative Example 3

In a nitrogen atmosphere, 10 g of hydroxyethyl cellulose (average degree of substitution of hydroxyethyl groups: 1.8 to 2.3) was added into 100 g of dimethyl sulfoxide and agitated and dissolved at 80°C for 4 hours. After the dissolution, 30 g of pyridine was added. To this, 40 g of stearoyl chloride was added, then allowed to react at 80°C for 8 hours, purified by allowing the resultant to precipitate in methanol, and dried. Thus, a cellulose derivative in which stearoyl groups were present as substituents on oxygen atoms in the hydroxyethyl cellulose was prepared. The resulting cellulose derivative was added to isododecane, and thus a cellulose derivative liquid mixture (mixed proportion: 10 mass%) was prepared.

### Evaluation

The cellulose derivatives and the cellulose derivative solution, the swollen gel of the cellulose derivative, or the liquid mixture prepared or used in Examples and Comparative examples were evaluated as follows. The results are presented in tables.

### (1) Average Degree of Substitution

¹H-NMR analysis was performed for each of the cellulose derivatives produced in Examples and Comparative Examples. Protons of a terminal methyl group of an acyl group derived from a fatty acid ester having 6 or more carbons in an esterified cellulose derivative appears around 0.9 ppm, and seven protons of a cellulose backbone appears around 3.2 to 5.2 ppm. Based on this content, the average degree of substitution of the introduced acyl group derived from the fatty acid ester was calculated from the calculated integrated value. Furthermore, the average degree of substitution of the acetyl group was calculated similarly based on methyl protons derived from the acetyl group that appeared around 2 ppm. Note that, in Examples 9 and 10, because it was difficult to clearly read the integrated value of protons of the cellulose backbone, the following benzoylation reaction was further performed to substitute for remaining hydroxy groups, and for the resulting cellulose derivative, calculation was performed based on the ratio of the integrated values of protons derived from the benzoyl group, protons derived from the ether bond-containing group, and protons derived from the lauroyl group, taking the average degree of substitution of all the substituents including the benzoyl group as 3.0.

### Benzoylation Reaction

100 mg of the cellulose derivative produced in Example 9 or 10 and 4 g of pyridine were added in a test tube, heated to 115°C under nitrogen, and dissolved. To this, 100 mg, which was an excessive amount, of benzoyl chloride was added dropwise and reacted at 115°C for 5 hours. Then, the reaction solution was added dropwise in 15 g of methanol, which was a poor solvent, to reprecipitate and purify, then the precipitate was dried, resulting in the formation of a cellulose derivative in which unreacted hydroxy groups were subjected to benzoylation.

### (2) Affinity

For the cellulose derivative solution, the swollen gel, or the liquid mixture produced in Examples and Comparative Examples, affinity of the cellulose derivative for an oil agent was evaluated based on the following criteria.
A: Dissolved by heating at 70°C
B: Became swollen gel by heating at 70°C
C: Dissolved by heating at 100°C
D: Was not dissolved or dispersed even by heating at 100°C and did not form swollen gel

### (3) Film Formability

The cellulose derivative solution, the swollen gel, or the liquid mixture produced in Examples and Comparative Examples was spread in an aluminum cup by using a spatula in a manner that the thickness became approximately 3 mm, and heated at 70°C for 1 hour. Furthermore, the cellulose derivative solution, the swollen gel, or the liquid mixture obtained in Examples and Comparative Examples was thinly spread on a glass plate by a spatula and heated at 70°C for 5 minutes to allow the solvent to be volatilized. Then, cooling to room temperature was performed. The condition of the film formed on each of the aluminum cup and the glass was visually observed and evaluated based on the following criteria.
A: Transparent films were formed on both the aluminum cup and the glass plate.
B: Films were formed on both the aluminum cup and the glass plate, but the colors of the films were semi-transparent or white.
C: Films were formed on both the aluminum cup and the glass plate, but the film had a crack.
D: Films were formed on both the aluminum cup and the glass plate, but the films were thicker than those of A to C (thin spreading was not possible).
E: No films were formed on the aluminum cup and the glass plate.

### [Table 1]

**Table 1**

| | Example 1 | Example 2 | Example 3 | Example 4 | Example 5 | Example 6 | Example 7 | Example 8 |
|---|---|---|---|---|---|---|---|---|
| Substituent | 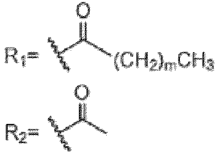 | | | | | | 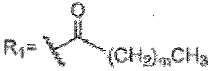 | |
| m | 10 | 12 | 14 | 16 | 16 | 16 | 6 | 10 |
| R₁ average degree of substitution | 2.3 | 2.2 | 2.1 | 1.9 | 2.1 | 2.4 | 2.8 | 2.8 |
| R₂ average degree of substitution | 0.2 | 0.2 | 0.2 | 0.2 | 0.2 | 0.4 | - | - |
| Total average degree of substitution | 2.5 | 2.4 | 2.3 | 2.1 | 2.3 | 2.7 | 2.8 | 2.8 |
| Affinity | B | B | B | B | B | A | A | A |
| Film formability | B | C | C | C | C | A | A | A |

**Table 2**

| | Example 9 | Example 10 | Example 11 | Comparative Example 1 | Comparative Example 2 | Comparative Example 3 |
|---|---|---|---|---|---|---|
| Substituent | 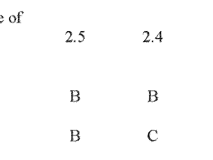 | | 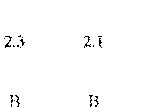 | 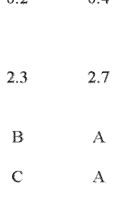 | 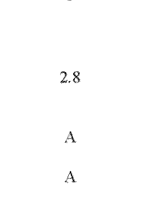 | 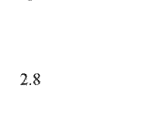 * R₁ bonded to O of HEC |
| m | 10 | 10 | 10 | - | 10 | 16 |
| R₁ average degree of substitution | 1.8 | 1.5 | 2.3 | 2.4 | 1.0 | 3.0 |
| R₂ average degree of substitution | 1.1 | 1.4 | 0.2 | - | 0.1 | - |
| Total average degree of substitution | 2.9 | 2.9 | 2.5 | 2.4 | 1.1 | 3.0 |
| Affinity | A | C | A | D | D | D |
| Film formability | A | D | B | E | E | E |

As shown in Tables 1 and 2, the cellulose derivative solution or the swollen gel of Examples had excellent affinity of the cellulose derivative for oil agents, the cellulose derivative dissolved in isododecane or isotridecyl isononanoate or formed swollen gel at 70 to 100°C and exhibited excellent affinity for oil agents, which were a hydrocarbon oil alone and an ester oil alone. Furthermore, the cellulose derivative solution of Examples also had excellent film formability. In particular, superior affinity resulted in superior film formability. Furthermore, when formation of a thin film was easy, spreading during application was good, and texture after the application was good. Meanwhile, in a case where no monovalent organic group (L^{a}) was contained or where the average degree of substitution was less than 1.1 (Comparative Examples 1 and 2) or in a case of a cellulose derivative in which an organic group was introduced into a hydroxyalkyl cellulose (Comparative Example 3), dissolution or dispersion in isododecane did not occur, no swollen gel was formed, and film formability was poor.

### Example 12

### Preparation of Liquid Foundation

Components shown in Table 3 were mixed, then stirred sufficiently, and the mixture was filled into a container to prepare liquid foundation. As a result of application of a small amount of the resulting liquid foundation on skin, the liquid foundation exhibited water resistance and sebum resistance and exhibited softness that was free from film-like texture.

### [Table 3]

**Table 3**

| Component | mass% |
|---|---|
| Cyclopentasiloxane | 12.5 |
| Mineral oil | 5.0 |
| Ethylhexyl methoxycinnamate | 4.0 |
| Isononyl isononanoate | 3.0 |
| Product name "Bentone Gel VS-5 PC V HV" (available from Elementis) | 3.0 |
| Phytosteryl macadamiate | 0.3 |
| Cellulose derivative solution of Example 1 | 3.0 |
| PEG-10 dimethicone | 1.5 |
| Product name "PolyAquol OS2" (available from Innovacos) | 1.0 |
| Product name "SDL-Ti70" (available from Daito Kasei Kogyo Co., Ltd.) | 12.3 |
| A mixture of product name "SDL-IOY50", product name "SDL-IOR50", and product name "SDL-IOB50" (available from Daito Kasei Kogyo Co., Ltd.) | 3.0 |
| Cellulose acetate | 3.0 |
| 1,3-Butylene glycol | 6.0 |
| Phenoxyethanol | 0.3 |
| Sodium chloride | 1.0 |
| EDTA-2Na | 0.03 |
| Purified water | balance |
| Total | 100.0 |

### Example 13

### Preparation of Sunscreen

Components shown in Table 4 were mixed, then stirred sufficiently, and the mixture was filled into a container to prepare a sunscreen. As a result of application of the resulting sunscreen on skin, texture was good, and a waterproofing characteristic was achieved.

### [Table 4]

**Table 4**

| Component | mass% |
|---|---|
| Diethylamino hydroxybenzoyl hexyl benzoate | 2.0 |
| Bis-ethylhexyloxyphenol methoxyphenyl triazine | 0.5 |
| Product name "Uvinul MC80" (available from BASF) | 7.0 |
| Diisopropyl sebacate | 10.0 |
| Dimethicone | 2.0 |
| Isododecane | 16.47 |
| Cellulose derivative solution of Example 2 | 10.0 |
| PEG-9 polydimethylsiloxyethyl dimethicone | 2.0 |
| Product name "DIS-OP-10A" (available from Sakai Chemical Industry Co., Ltd.) | 4.0 |
| Product name "DIF-OP-3W" (available from Sakai Chemical Industry Co., Ltd.) | 10.0 |
| Cellulose acetate | 5.0 |
| 1,3-Butylene glycol | 3.0 |
| Phenoxyethanol | 0.2 |
| Ethanol | 7.0 |
| Sodium chloride | 0.5 |
| EDTA-2Na | 0.03 |
| Purified water | balance |
| Total | 100.0 |

## Claims

1. A composition comprising a cellulose derivative and an oil agent,
the cellulose derivative containing a monovalent organic group (L^{a}) that substitutes for at least some of hydrogen atoms constituting hydroxy groups in cellulose, the monovalent organic group (L^{a}) containing a hydrocarbon group having 6 or more carbons at a terminal, and the monovalent organic group (L^{a}) containing no ether bond, and an average degree of substitution of the monovalent organic group (L^{a}) being 1.1 or greater.

2. The composition according to claim 1, wherein the oil agent is at least one selected from the group consisting of a hydrocarbon oil, an ester oil, and a silicone oil.

3. The composition according to claim 2, wherein a content of the hydrocarbon oil is greater than 80 mass%.

4. The composition according to claim 2, wherein a content of the ester oil is greater than 50 mass%.

5. The composition according to any one of claims 1 to 4, wherein the cellulose derivative contains a monovalent organic group (L^{c}) having 5 or less carbons, the monovalent organic group (L^{c}) substituting for at least some of hydrogen atoms constituting hydroxy groups in the cellulose, the monovalent organic group (L^{c}) containing a hydrocarbon group at a terminal, and the monovalent organic group (L^{c}) containing no ether bond.

6. The composition according to claim 5, wherein, in the cellulose derivative, the average degree of substitution of the monovalent organic group (L^{a}) is greater than or equal to an average degree of substitution of the monovalent organic group (L^{c}).

7. The composition according to claim 5 or 6, wherein, in the cellulose derivative, the average degree of substitution of the monovalent organic group (L^{c}) is 1.0 or less.

8. The composition according to any one of claims 1 to 7, wherein the monovalent organic group (L^{a}) is a group represented by Formula (1):
-X¹-R¹ (1)
where R¹ represents the hydrocarbon group having 6 or more carbons, X¹ represents a direct bond or a group capable of forming a linking group by bonding to an oxygen atom constituting a hydroxy group in the cellulose, and a bond extending to left bonds to an oxygen atom in a cellulose backbone.

9. An oil-based cosmetic material comprising the composition described in any one of claims 1 to 8.

10. A cellulose derivative comprising a monovalent organic group (L^{b}) containing a group represented by Formula (2) at a terminal:
-O-R² (2)
where R² represents a hydrocarbon group having 6 or less carbons,
the monovalent organic group (L^{b}) substituting for at least some of hydrogen atoms constituting hydroxy groups in cellulose.

11. The cellulose derivative according to claim 10, wherein the monovalent organic group (L^{b}) contains 2 or more ether bonds.

12. The cellulose derivative according to claim 10 or 11, wherein the monovalent organic group (L^{b}) bonds to the cellulose backbone through an ester bond having an oxygen atom constituting the hydroxy group as a constituent atom.

13. The cellulose derivative according to any one of claims 10 to 12, wherein the monovalent organic group (L^{b}) contains a (poly)oxyalkylene chain.

14. The cellulose derivative according to any one of claims 10 to 13, further comprising a monovalent organic group (L^{a}) substituting for at least some of hydrogen atoms constituting hydroxy groups in the cellulose, the monovalent organic group (L^{a}) containing a hydrocarbon group having 6 or more carbons at a terminal, and the monovalent organic group (L^{a}) containing no ether bond.

15. The cellulose derivative according to claim 14, wherein an average degree of substitution of the monovalent organic group (L^{a}) is greater than an average degree of substitution of the monovalent organic group (L^{b}).

16. A composition comprising the cellulose derivative described in any one of claims 10 to 15 and an oil agent.

17. An oil-based cosmetic material comprising the composition described in claim 16.
